# EUROPEAN PATENT SPECIFICATION

(11) **EP 1 765 317 B1**
(45) Date of publication and mention of the grant of the patent: **01.10.2008**
(21) Application number: 05772060.9
(22) Date of filing: 28.06.2005
(51) Int. Cl.: A61K 31/13, A23K 1/16, A23K 1/18

(54) **MATERIALS AND METHODS FOR IMPROVING SHELLFISH HEALTH, IMMUNITY AND GROWTH**
MATERIALIEN UND VERFAHREN ZUR VERBESSERUNG DER GESUNDHEIT, DER IMMUNITÄT UND DES WACHSTUMS BEI SCHALENTIEREN
MATIERES ET METHODES PERMETTANT D'AMELIORER LA SANTE, L'IMMUNITE ET LA CROISSANCE DES MOLLUSQUES ET DES CRUSTACES

(30) Priority: 30.06.2004 US 584574 P; 24.01.2005 US 646284 P
(43) Date of publication of application: 28.03.2007
(73) Proprietor: Walcom Animal Science (I.P.3) Limited, Kowloon, Hong Kong (CN)
(72) Inventor: CHI, Francis, Flat D, 11/F, Tower 10, Hong Kong (CN)
(74) Representative: Perry, Robert Edward
(86) International application number: PCT/EP2005/006948
(87) International publication number: WO 2006/002868

(56) References cited:
- WO-A-02/076391
- US-A- 5 698 246
- US-B1- 6 306 453
- SHIAU SHI-YEN ET AL: "Dietary pantothenic acid requirement of juvenile grass shrimp, Penaeus monodon" JOURNAL OF NUTRITION, vol. 129, no. 3, March 1999 (1999-03), pages 718-721, XP002348483 ISSN: 0022-3166
- REDDY H R V ET AL: "Evaluation of the dietary essentiality of vitamins for Penaeus monodon" AQUACULTURE NUTRITION, vol. 5, no. 4, December 1999 (1999-12), pages 267-275, XP002348484 ISSN: 1353-5773
- DATABASE BIOSIS [Online] BIOSCIENCES INFORMATION SERVICE, PHILADELPHIA, PA, US; December 2004 (2004-12), ZHU WEI ET AL: "Dietary pantothenic acid requirement of juvenile abalone, Haliotis discus hannai Ino" XP002351573 Database accession no. PREV200510022746 & JOURNAL OF SHELLFISH RESEARCH, vol. 23, no. 4, December 2004 (2004-12), pages 1045-1049, ISSN: 0730-8000

## Description

### Background of the Invention

The world's population is increasing rapidly, causing a concurrent increase in demand for seafood. Moreover, recent studies demonstrating the beneficial properties of shellfish have added to the demand (see Takezaki, T. et al., "Diet and lung cancer risk from a 14-year population-based prospective study in Japan: with special reference to fish consumption," Nutr Cancer, 45(2):160-7 (2003); and Su, X. et al., "Omega-3 polyunsaturated fatty acid content in different edible portions of commercial scallop," Asia Pac J Clin Nutr., 12 Supp1:S63 (2003)). A variety of methods have developed in attempts to bridge the gap between the supply and demand of seafood. For example, many different shellfish species are now produced in aquacultures. Some species are cultured during the complete life cycle whereas others are cultured from wild-harvested seed. Still others are raised in hatcheries and released to open water for later harvesting.

Unfortunately, the cost in time and operation for producing marketable sized shellfish using the methods described above is very high. For example, start-up costs alone for establishing an oyster farm in Alaska can begin in the tens of thousands and rapidly ratchet up to hundreds of thousands as the size of the farm increases.

The growth cycle for most shellfish is lengthy in time. For abalone and lobsters, for example, several years are often necessary for development and growth before obtaining a desirable size for consumption. Therefore, there is a need for shortening the growth cycle for shellfish and augmenting growth to more rapidly provide marketable shellfish to the consumer.

Diseases and contaminated water are a threat to any shellfish aquaculture, hatchery, or farm. Most diseases in shellfish are caused by opportunistic pathogens, *i.e.,* bacteria that cause disease in shellfish that are weakened or stressed. Microscopic marine organisms, such as the one-celled dinoflagellates that produce a toxin called paralytic shellfish poison (PSP), can accumulate in exposed shellfish and be harmful to the end-consumer.

Vaccines and antibiotics are two available methods used to protect shellfish against diseases. The use of drugs (*i.e.,* antibiotics, vaccines, etc.), which is expensive, has to be considerably reduced in aquaculture to avoid environmental hazards and the risk of the development of resistance. Therefore, there is a constant need for enhancing the immunogenicity of shellfish.

Cysteamine is a depleting agent of the growth inhibitor somatostatin, which can increase growth and longevity in vertebrates by promoting growth hormone secretion from the pituitary. However, shrimp are invertebrate animals, which have a different metabolism and endocrine system from that of the vertebrate animal. Thus, whether cysteamine can promote the growth of shrimp, let alone improve the health of shellfish, is still a question. Insofar as is known, cysteamine compounds have not been previously reported as being useful for improving the health of shellfish.

### Brief Summary of the Invention

The subj ect invention provides materials and methods for improving the health of shellfish. In particular, the present invention concerns materials and methods for: accelerating and/or augmenting growth in shellfish; improving immunity to diseases and other contaminants; enhancing fertility; and/or increasing the success of larval production and survival.

The subject invention provides methods for improving the health of shellfish through the administration of a cysteamine compound to the shellfish. Specifically, an effective amount of a cysteamine compound is introduced to shellfish to promote shellfish health, growth, and population numbers. For example, cysteamine, or various cysteamine salts, prodrugs, analogs, derivatives, conjugates, and metabolites, are administered to shellfish.

In one embodiment of the invention, a composition comprising a cysteamine compound is introduced into water in which shellfish are harbored. In a related embodiment, the composition comprises additional agents that are useful in promoting shellfish health. For example, antibiotics and/or vaccines may be concurrently administered with a cysteamine compound to shellfish.

The method and composition of the invention are useful for treating shellfish during any stage of development to improve health. A cysteamine compound is preferably administered to shellfish by introducing the cysteamine compound into water that contains or will contain the shellfish to be treated.

### Brief Description of Drawings

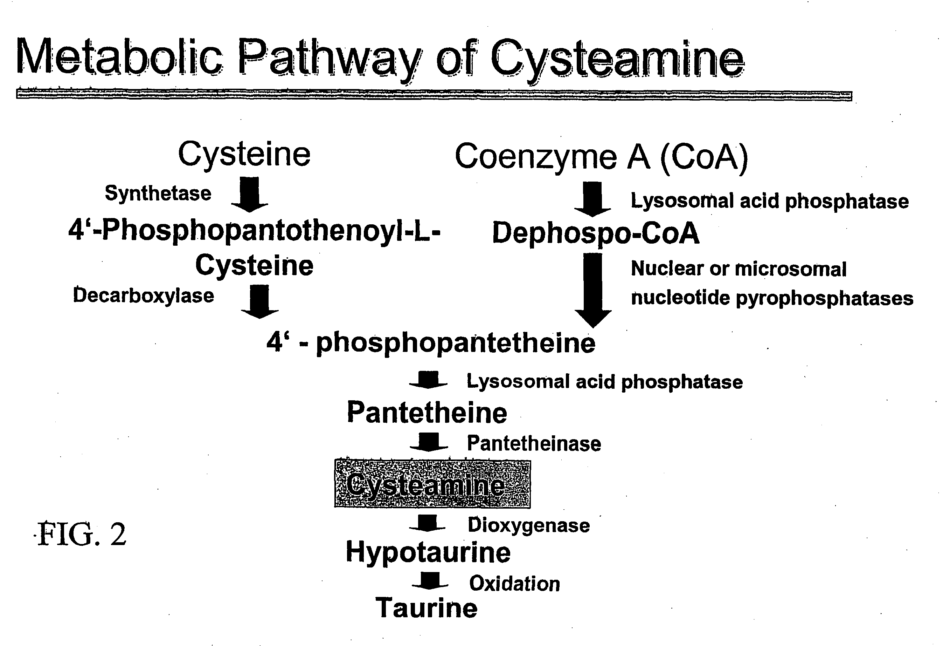

**Figure 2** shows a metabolic pathway of cysteamine.

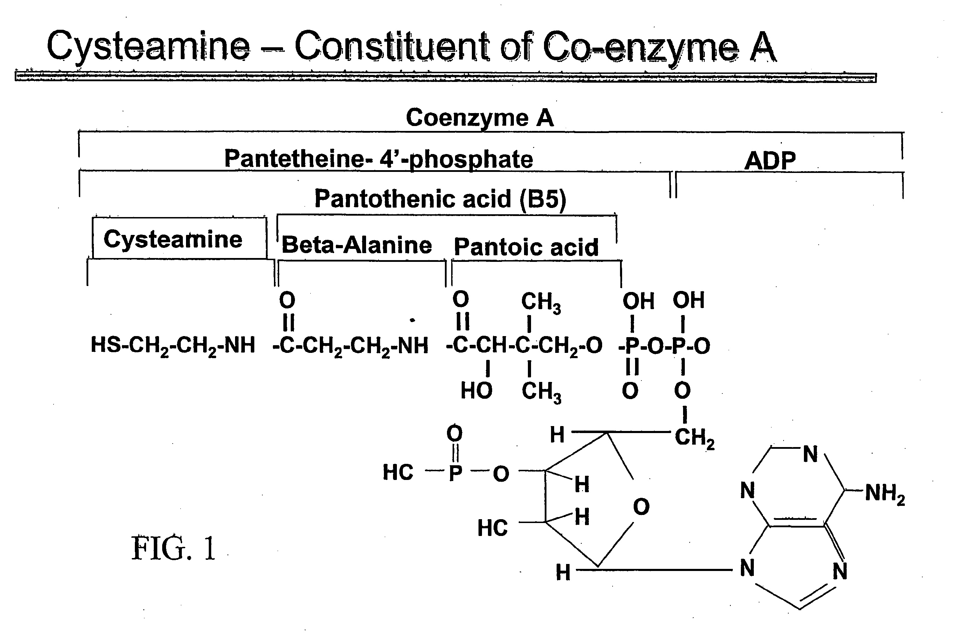

**Figure 1** shows cysteamine as a constituent of co-enzyme A.

### Detailed Disclosure of the Invention

The subject invention provides unique materials and methods for improving the health of shellfish. Specifically, the subject invention provides materials and methods for accelerating and/or augmenting growth in shellfish; improving immunity to diseases and other contaminants; enhancing fertility; and/or increasing the success of larval production and survival.

In particular, the invention concerns administering a cysteamine compound to shellfish, in an amount effective to promote shellfish health, growth, and population numbers. One embodiment of the invention is a composition for improving shellfish health, wherein the composition comprises a cysteamine compound. Preferably, the composition is an aqueous mixture or an aqueous emulsion including the cysteamine compound. More preferably, a cysteamine compound (*i*.*e*., cysteamine hydrochloride) is provided in solid feed such as sinking or floating feed for shellfish.

As used herein, the term "shellfish" refers to all non-fish aquatic life. As contemplated herein, shellfish includes aquatic invertebrates having a soft, unsegmented body that can be enclosed in a shell. For example, references to shellfish include crustaceans such as prawns, shrimp, crawfish, crayfish, crabs, lobsters; and mollusks such as abalone, clams, mussels, oysters, scallops, octopi, squid, and snails. References to shellfish herein can also include turtles, sea urchins, and sea cucumbers as well as invertebrates that lack a shell (*i.e.,* jellyfish).

The term "shellfish health," as used herein, generally refers to a variety of parameters that affect the overall condition of a shellfish. Specific parameters upon which shellfish health is based include: the size (or growth) of a shellfish; the length of time in a growing cycle; the immune system's ability to adequately address exposure to diseases and contamination; and the ability to reproduce offspring. As contemplated herein, improving shellfish health includes reducing shellfish mortality; increasing antibody titer/lymphocyte number; and increasing cytokine secretion.

"Concurrent administration" and "concurrently administering," as used herein, includes administering a compound or method suitable for use with the methods of the invention (administration of a cysteamine compound) to improve shellfish health. For example, an antibiotic and/or vaccine can be administered concurrently with the materials and methods of the invention to improve shellfish health.

According to the subject invention, a compound can be provided in admixture with a cysteamine compound, such as in an aqueous emulsion; or the compound and cysteamine can be provided as separate compounds, such as, for example, separate compositions administered consecutively, simultaneously, or at different times. Preferably, if the cysteamine compound and the known agent (or therapeutic method) for improving shellfish health are administered separately, they are not administered so distant in time from each other that the cysteamine compound and the known agent (method) cannot interact.

Contemplated compounds that can be concurrently administered with a cysteamine compound of the invention include, but are not limited to, Microbicides such as Formalin, Albendazole, Cypermethrin, Deltamethrin, Hydrogen peroxide, and Teflubenzuron; Antimicrobials such as Oxytetracycline, Alkyltrimthylammonium calciumoxytetracycline, Bicozamycin benzoate, cyanphenicol, Doxycycline, Florofenicol, Josamycin, Kitasamycin, Lincomycin, Myroxacin, Nalidixic acid, Phosphomycin, Spiramycin, Sulfadimethoxine-ormetoprim, and Sulfamerazine; and Vaccines such as the *Vibrio parahaemolyticus* (Vibrogen-S) vaccine, Penaeid multivalent bacterin (P.M.B. vaccine), and *Vibrio* sp. bacterin.

The advantages of cysteamine, as set forth herein, may be achieved in shellfish by promoting the endogenous production of cysteamine through natural metabolic processes such as those observed in mammals (*i.e*., through the action of co-enzyme A or as a metabolite of cysteine (see Figures 1 and 2 of mammalian production of cysteamine)). This may be achieved by, for example, the administration of pantothenic acid to shellfish.

The term "effective amount," as used herein, refers to the amount necessary to elicit the desired biological response. In accordance with the subject invention, the effective amount of a cysteamine compound is the amount necessary to improve shellfish health. In a preferred embodiment, the effective amount of a cysteamine compound is the amount necessary to accelerate and/or augment growth in shellfish; improve immune response to diseases and other contaminants; enhance fertility; and/or increase the success of larval production and survival. For example, the improvement in shellfish health can be a 1%, 5%, 10%, 15%, 20%, 25%, 30%, 35%, 40%, 45%, 50%, 55%, 60%, 65%, 70%, 75%, 80%, 85%, 90%, 95%, 100%, 150%, 200%, 250%, or 300% acceleration and/or augmentation in growth; improvement in immunity response to a disease and/or contaminant; and/or enhancement in fertility. More specifically, shellfish health is improved as a result of reduced shellfish mortality; increased antibody titer/lymphocyte numbers; and increased cytokine secretion.

### Accelerated and/or Augmented Growth

In one embodiment of the invention, a cysteamine compound is administered to shellfish to accelerate and/or augment growth. As contemplated herein, to "accelerate and/or augment growth" refers to the ability to shorten developmental periods during a normal growth cycle and/or increase the overall size of the shellfish.

For example, with oysters, the materials and methods of the invention could shorten the length of time for a normal growth cycle from around 2 years to around 6 months to 1.8 years to reach marketable size. Or, the invention can increase the overall size of an oyster from a cocktail oyster size of 3,81 cm (1.5 inches) to an appetizer oyster size of about 6,35 cm (2.5 inches). In certain embodiments, the subject materials and methods of the invention both accelerate and augment growth (*i.e*., decrease the length of normal growth cycle and increase overall size of the shellfish).

In another example, the growth cycle of mussels grown in water temperatures below 18,3° C (65° F) takes normally about 2 years to reach market size. By using the materials and methods of the invention, the growth cycle for such shellfish can be decreased from about 2 years to about 6 months to 1.8 years to reach market size.

### Improving Immune Response

In another embodiment, a cysteamine compound is administered to shellfish to improve immunity response to diseases and other contaminants. As contemplated herein, "improving immunity" refers to boosting the shellfish immune system to more effectively attack harmful microorganisms and/or contaminants and heal the shellfish of any damages incurred by exposure to such microorganisms and/or contaminants. By improving immunity, the subject invention also increases the likelihood of success in larval production and survival.

According to the subject invention, introduction of a cysteamine compound to shellfish can: (1) proactively augment shellfish immunity to promote resistance to disease and/or contamination; and/or (2) treat and promote rapid recovery from current exposure to harmful microorganisms and/or contamination.

As contemplated herein, the subject invention improves shellfish immune response to a variety of microorganisms and contaminants. For example, the subject invention improves oyster immune response to a variety of diseases and pathogens including, without limitation, Oyster Velar Virus Disease (OVVD); Gill Disease of Portuguese Oysters; Haemocytic Infection Virus Disease of Oysters; Herpes-Type Virus Disease of Oysters; Extracellular Giant "Rickettsiae" of Oysters; *Perkinsus marinus* ("Dermo" Disease) of Oysters; *Perkinsus* sp. of European Flat Oysters; Kidney Coccidia of Oysters; *Minchinia armoricana* of Oysters; Marteiliosis (Aber disease) of Oysters; *Marteilioides chungmuensis* of Oysters; *Bonamia ostreae* of Oysters; Oyster Egg Disease; Invasive Ciliates of Juvenile Oysters; *Mytilicola intestinalis* (Red Worm Disease) of Oysters; Haemocytic Neoplasia of Oysters; Juvenile Disease of Eastern Oysters; Viral Gametocytic Hypertrophy of Oysters; Nocardiosis of Oysters; *Mikrocytos machine* (Denman Island Disease) of Oysters; *Haplosporidium nelsoni* (MSX) of Oysters; *Haplosporidium costale* (SSO) of Oysters; *Ostracoblabe implexa* (Shell Disease) of Oysters; Oyster Trematode Diseases; *Mytilicola orientalis* (Red Worm) of Oysters; Parasite Copepods on Oyster Gills; Pea Crabs in Oysters; Malpeque Disease of Oysters; Papova-Like Virus Infection of Pearl Oysters; Apicomplexan Parasite of New Zealand Oysters; *Haplosporidium* sp. of Pearl Oysters; *Marteilia sydneyi* of Oysters; *Marteilioides branchialis* of Oysters; *Bonamia exitiosus* (Bonamiasis of New Zealand Dredge Oysters); *Bonamia* sp. (Bonamiasis of Australian Oysters); *Mikrocytos roughleyi* (Australian Winter Disease) of Oysters; Microsporidiosis of Dredge Oysters; *Rikettsia-like* and *Chlamydia-like* Organisms of Oysters; *Vibrio* spp. (Larval and Juvenile Vibriosis) of Oysters; Hinge Ligament Disease of Juvenile Oysters; Digestive Tract Impaction of Larval Oysters; Gregarine Parasitism of Oysters; Hexamitiasis of Oysters; *Ancistrocoma*-like Ciliates of Oysters; *Sphenophyra*-like Ciliates of Oysters; Gill Trichodinids of Oysters; *Sirolpidium zoophthorum* (Larval Mycosis) of Oysters; Oyster Gill Turbellaria; Nematode Parasitism of Oysters; Shell-boring Polychaetes of Oysters; Shell-burroing Sponges of Oysters; and Pyramidellid Snails of Oysters.

In another example, the subject invention can improve mussel immune response to a variety of diseases and pathogens including, without limitation, Virus-like Diseases of Mussels; Haplosporidian Infection of Mussels; *Marteilia refringens*/*maurini* of Mussels; *Steinhausia mytilovum* (Mussel Egg Disease); Phototrophic Endolity Invasion of Mussel Shells; *Proctoeces maculates* Trematode Disease of Mussels; Mussel Gill Turbellaria; *Mytilicola intestinalis* (Red Worm Disease) of Mussels; Kidney Coccidia of Mussels; Bucephalid Trematode Diseases of Mussels; *Mytilicola orientalis* (Red Worm) of Mussels; Pea Crabs in Mussels; Haemocytic Neoplasia of Mussels; *Mytilicola porrecta* (Red Worm) of Mussels; *Rickettsia*-like and *Chlamydia*-like Organisms of Mussels; Gregarine Parasitism of Mussels; Intracellular Ciliates of Mussels; *Phenophyra*-like Ciliates of Mussels; *Ancistrum mytili* Gill Ciliate of Mussels; Mycotic Periostracal Sloughing of Mussels; Trematode Metacercariae of Mussels; Parasitic copepods on Mussel Gills; Shell-boring Polychaetes of Mussels; and Shell-burrowing Sponges of Mussels.

In yet another example, the subject invention can improve clam and cockle immune response to a variety of diseases and pathogens including, without limitation, Viral infections of Clams; Brown Ring Disease of Manila Clams; *Perkinsus* of Clams and Cockles; Haplosporidian Infection of Clams; Microsporidiosis of Clams; Amoeboflagellate Disease of Larval Geoduck Clams; *Mytilicola inestinalis* (Red Worm Disease) of Clams and Cockles; Nuclear Inclusion X (NIX) of Pacific Razor Clams; Kidney Coccidia of Clams; QPX (quahog parasite unknown) of Clams; *Mytilicola orientalis* (Red Worm) of Clams and Cockles; Pea Crabs in Clams and Cockles; Haemocytic Neoplasia of Clams; Gonadal Neoplasia of Clams; Endonucleobiotic Bacteria of Clams in Portugal; Mycoplasma-like Infection of Cockles; Cryptosporidiosis of Clams; *Marteilia*-like Parasite of Giant Clams; Amoebiasis of Cockles; *Rickettsia*-like and *Chlamydia-*like Organisms of Clams and Cockles; *Vibrio* spp. (Larval and Juvenile Vibriosis) of Clams; Hinge Ligament Disease of Juvenile Clams; Gregarine Parasitism of Clams and Cockles; *Sphenophyra*-like Ciliates of Clams and Cockles, *Ancistrocoma pelseneeri* and *A. myae* Ciliates of Clams; Gill Trichodina of Clams and Cockles; *Sirolpidium zoophthorum* (Larval Mycosis) of Clams; Turbellaria of Clams; Trematode Metacercariae of Clams and Cockles; Shell-boring Polychaetes of Clams; and Siphon Snails of Clams and Cockles.

In a further example, the subject invention can improve scallop immune response to a variety of diseases and pathogens including, without limitation, *Perkinsus* sp. of Japanese Scallops in Asia; Scallop Haplosporidian; *Marteilia* sp. of Scallops; Brood-;pouch Copepod of Scallop Gills; Pea Crabs in Scallops; Intracelllular Bacterial Disease of Scallops; Bacterial Abscess Lesions of Scallops; *Perkinsus qugwadi* (SPX) of Scallops; Kidny Coccidia of Scallops; *Perdinsus karlssoni* of Scallops; Scallop Protistan G; Microsporidiosis of Scallops; Trematode Metacercariae of Scallops; Virus-like Infection of Scallops; Chlamydiosis of Scallops; *Rickettsia*-like and *Chlamydia*-like Organisms of Scallops; *Vibrio* spp. (Larval Vibriosis) of Scallops; Gregarine Parasitism of Scallops; Gill Trichodinids of Scallops; Scallop Gill Turbellaria; Nematode Parastitism of Scallops; Shell-boring Polychaetes of Scallops; and Shell-burrowing Sponges of Scallops.

In another example, the subject invention can improve abalone immune response to a variety of diseases and pathogens including, without limitation, Kidney Coccidia of Abalone; Sabellid Polychaete Infestation of Disease in Abalone; *Labyrinthuloides haliotidis* of Abalone; Amyotrophia of Abalone; Blister Disease of Cultured Abalone; Withering Syndrome of Abalone; *Perkinsus olseni* of Abalone; Haplosporidian parasite of Abalone; Fungal Disease of Abalone; Nematode Parasitism of Abalone; Bacterial Diseases of Abalone; Ciliates Associated with Abalone; Trematode Metacercariae of Abalone; and Shell-borring Polychaetes of Abalone.

In yet another example, the subject invention can improve sea urchin immune response to a variety of diseases and pathogens including, without limitation, Namatode Parasitism of Sea Urchin; Bald-Sea-Urchin Disease; *Paramoeba invadens* of Sea Urchins; Spotted gonad Disease of Sea Urchins; Black Sea Urchin Plage; Trematode Metacercariae of Sea Urchins; and Turbellarian Parasitism of Sea Urchins.

In yet another example, the subject invention can improve lobster immune response to a variety of diseases and pathogens including, without limitation, *Paramoeba perniciosa* (Paramoebiasis) of Lobsters; Gaffkemia of Lobsters; *Anophryoides haemophila* (Ciliate Disease) of Lobsters;*Pseudocarcinonemetes homari* of Lobsters; Microsporidosis of Lobsters; *Hematodinium* sp. of Norway Lobster; *Carcinonemertes australiensis* of Lobsters; Parasitic Copepods of Lobsters; *vibrio* spp. (Juvenile Vibriosis) of Lobsters; Chitinolytic Bacterial Shell Disease of Lobsters; Gregarine Parasitism of Lobsters; *Lagenidium* sp. (Fungus Disease) of Lobsters; *Fusarium* sp. (Fungus or Burn Spot Disease) of Lobsters; *Haliphthoros* sp. (Fungus Disease) of Lobsters; Nematodes in Lobsters; Trematode Metacercariae in Lobsters; and Acanthocephalan Larvae in Lobsters.

In yet another example, the subject invention can improve shrimp and prawn immune response to a variety of diseases and pathogens including, without limitation, Rickettsia-like Infection of Pandalid Shrimp; Protistan Pathogen of Pandalid Shrimp (SPP); *Sylon* (Rhizocephalan Disease) of Shrimp and Prawns; *Baculovirus penaei* (BP Virus Disease) of Penaeid Shrimp; Monodon Baculovirus (MBV) Disease of Penaeid Shrimp; Baculoviral Midgut-gland Necrosis (BMN) of Penaeid Shrimp; White Spot Syndrome Baculovirus Complex of Penaeid Shrimp; Hepatopancreatic Parvovirus (HPV) Disease of Shrimp and Prawns; Infectious Hypodermal and Haematopoietic Necrosis Virus (IHHNV) of Penaeid Shrimp; Lymphoidal Parvo-like Virus Disease of Penaeid Shrimp; Lymphoid Organ Vacuolization Virus (LOVV) of Penaeid Shrimp; Reo-like Virus (REO) Disease of Penaeid Shrimp; Taura Syndrome Virus of Penaeid Shrimp; Rhabdovirus Disease of American Penaeid Shrimp; Yellow-head Virus Disease (YHD) of Penaeid Shrimp; Rickettsial Infection of Penaeid Shrimp; Necrotizing Hepatopancreatic of Penaeid Shrimp; Mycobacteriosis of Penaeid Shrimp; *Vibrio penaeicida* of Cultured Kurama Prawns; Larval Bacterial Necrosis of Freshwater Shrimp; Haplosporidian Infections of Penaeid Shrimp; Gregarine Disease of Penaeid Shrimp; Ciliate Disease of Penaeid Shrim; Gut and Nerve Syndrom (GNS) of Penaeid Shrimp; Larval Mid-cycle Disease (MCD) of Freshwater Shrimp; Red Disease of Penaeid Shrimp; *Vibrio* spp. (*Vibrio* Disease) of Cultured Shrimp; Chitinolytic Bacterial Shell Disease of Shrimp and Prawns; Filamentous Bacterial Disease of Shrimp and Prawns; Microsporidosis (Cotton shrimp Disease) of Shrimp and Prawns; Larval Mycosis of Shrimp and Prawns; *Fusarium* sp. (Fungus Disease) of Shrimp and Prawns; Nematomorph Parasitism of Pandalid Shrimp; and Black Gill Syndrome of Shrimp and Prawns.

In yet another example, the subject invention can improve crab immune response to a variety of diseases and pathogens including, without limitation, Viral Diseases of Crabs; Rickettsia and Chlamydia of Crabs; Haplosporidosis of Grabs; *Paramoeba perniciosa* (Grey Crab Disease); *Hematodinium perezi* and *Hematodinium* sp. of Atlantic Crabs; Chitinolytic Fungal Disease (Black Mat Syndrome) of Crabs; *Carcinonemertes* spp. of Crabs; *Mesanophrys* spp. (Ciliate Disease) of Crabs; *Hematodinium* sp. (Bitter Crab Disease); Rhizocephalan Parasites of Crabs; *Hematodinium* spp. of Crabs in Australia; Chitinolytic Bacterial Shell Disease of Crabs; Microsporidosis of Crabs; Trematode Metacercariae of Crabs; Acanthocephalan Parasitism of Crabs; Nematomorph Parasitism of Crabs; and *Lagendium* spp. (Fungus Disease) of Crabs.

In yet another example, the subject invention can improve crayfish immune response to a variety of diseases and pathogens including, without limitation, *Psorospermium* spp. (Protozoan Disease) of European Crayfish; Therlohaniasis of Crayfish; Burn Spot Disease (Fungus Disease) of Crayfish; Crayfish Plague (Fungus Disease); Baculovirus of Blue Crayfish; Rickettsia of Crayfish; *Nocardia* sp. (Bacterial Disease) Crayfish; *Proteus* or *Pseudomonas* Bacterial Septicaemia of Crayfish; Chitinolytic Bacterial Shell Disease of Crayfish; *Psorospermium* sp. (Protozoan Infection) of American Crayfish; *Saprolegnia* spp. (Fungus Disease) of Crayfish; Trematodes in Crayfish; Turbellaria Infestation of Crayfish; and Branchiobdellida Annelid Parasitism of Crayfish.

### Enhancing Fertility

According to the subject invention, a cysteamine compound is administered to shellfish to enhance fertility. As contemplated herein, to "enhance fertility" refers to the ability to maximize fertilization of shellfish. In one embodiment of the subject invention, a cysteamine compound is administered to shellfish to manipulate sexual development. Sexual development manipulation can include increasing the number of eggs and sperm that are produced and discharged by shellfish or shortening the time to which shellfish are capable of reproduction.

Administration of a cysteamine compound to shellfish, in accordance with the subject invention, can be accomplished by any suitable method and technique presently or prospectively known to those skilled in the art. Specifically exemplified herein is the introduction of a cysteamine compound, either alone or concurrently with additional compound(s) or method(s), into water containing the shellfish to be treated. The cysteamine compound can be introduced as a composition, in any available form including in a liquid (*i.e.,* solvent, oil), in an aqueous mixture, in an aqueous emulsion, in a solid carrier or substrate, or other vehicles provided the vehicles are compatible with the administration of the cysteamine compound into water harboring the shellfish to be treated, and do not adversely affect the shellfish.

A variety of suitable adjuvants may also be used in compositions comprising a cysteamine compound. For example, emulsifiers, antifoaming agents (or defoaming agents), antioxidants, preservatives, coloring agents, and the like can be included in compositions of the invention. In one embodiment, the adjuvants are present in compositions of the invention in minor amounts, *i.e.,* less than about. 5% by volume, and preferably, less than 1% by volume. In other embodiments, greater amounts of adjuvants are present in compositions of the invention, *i.e.,* up to 70% by volume. All such adjuvants should be noninjurious and nontoxic to shellfish being treated.

According to the present invention, suitable emulsifiers (*i.e*., surfactants or dispersants) can be cationic, anionic, nonionic, or amphoteric emulsifiers. Preferred emulsifiers include, for example, food grade emulsifiers which are widely available. An overview of some types of suitable emulsifiers for use with the invention include those set forth in A. J. St. Angelo, "A Brief Introduction to Food Emulsion and Emulsifiers," at pp. 1-8 of G. Charalambous et al., Eds., Food Emulsifiers-Chemistry, Technology, Functional Properties and Applications, Elsevier Science Publishing Co. Inc., New York, N.Y. (1989).

Where needed, after the introduction of a cysteamine compound to water in which shellfish are harbored, a metering or mixing pump, or an inline mixer (*i.e*., a mixing valve, nozzle or orifice), an aerator, or other device known to the skilled artisan may be used to accomplish the direct dispersion of the cysteamine compound in water.

In one embodiment, an aqueous mixture, emulsion, or dispersion including a cysteamine compound is introduced into water harboring shellfish to be treated. The aqueous mixture, emulsion, or dispersion of the invention can contain from about 0.1% to about 95% of a cysteamine compound, wherein all percentages being by volume, based on the final volume of the composition. The composition can be further diluted when added to the water environment containing the shellfish to be treated according to the present invention. The amount of cysteamine compound used can be varied based upon the health (*i.e*., size, age, *etc*.) of the shellfish to be treated.

In a preferred embodiment, a solid feed mixture (*i.e.,* sinking or floating feed) including a cysteamine compound is introduced to shellfish to be treated. The feed mixture of the invention can contain from about 0.1% to about 95% of a cysteamine compound, wherein all percentages being by volume, based on the final volume of the composition. The amount of cysteamine compound used can be varied based upon the health (*i.e*., size, age, *etc.)* of the shellfish to be treated.

The cysteamine compounds of the subject invention can be formulated according to known methods for preparing compositions for use in administration to shellfish. The formulations may be presented in unit-dose or multi-dose containers, for example sealed ampoules and vials, and may be stored in a freeze dried (lyophilized) condition requiring only the condition of the sterile liquid carrier, for example, water, prior to use. Extemporaneous solutions and suspensions may be prepared from sterile powder, granules, tablets, *etc.* It should be understood that in addition to the ingredients particularly mentioned above, the formulations of the subject invention can include other agents conventional in the art having regard to the type of formulation in question.

In certain methods of the invention, the effective amount of cysteamine introduced is dependent on factors such as water pH, hardness, alkalinity, temperature, and the like.

The shellfish that are treated according to the invention include those that are held in a confined body of water, such as a shipping container, holding tank, aquarium, pool, or small pond, large body of water and those that are found in unconfined water, such as streams or off of a beach.

Following is an example that illustrates a procedure for practicing the invention. This example should not be construed as limiting. All percentages are by weight and all solvent mixture proportions are by volume unless otherwise noted.

### Example 1-Shrimp

AQUANIN is a micro-granule, produced by the Walcom Bio-Chemical Company, Shanghai, China, 30% of which is comprised of cysteamine hydrochloride as an active ingredient for livestock application. Example 1 describes the effect of AQUANIN on juvenile shrimp (*Penaeus vannamei*), in particular the effect of cysteamine hydrochloride on shrimp growth in different dosages.

Juvenile shrimp (*Penaeus vannamei*) with average body weight 0.43-0.49g were used in this example. The juvenile shrimp were fed standard shrimp feed provided from Guangdong ZhanJiang HengXing company in Guangdong, China. The shrimp feed was composed of the following products listed in Table 1.

| Table 1—Shrimp Feed | |
|---|---|
| Fishmeal | 35% |
| Fermented soybean meal | 28% |
| Peanut meal | 5% |
| Wheat flour | 24.885% |
| Phosphor fat meal | 2% |
| Fish oil | 1% |
| Dicalcium phosphate | 1% |
| Maltose | 2% |
| Vitamin premix | 0.1% |
| Mineral premix | 0.1% |
| Choline Chloride | 0.5% |
| Antioxidant | 0.015% |

The trial experiment of Example 1 was conducted for 42 days. A total of 30 tails of juvenile shrimp with average body weights of 0.43-0.49g were randomly divided into 4 groups (G1, G2, G3 and a Control group), each group contained 3 replicates, each replicate used one aquarium (size: 0.8X0.6X0.6 meters), with sea water circulation. The G1 group was fed 500 ppm AQUANIN (150 ppm of cysteamine hydrochloride) and standard shrimp feed. The G2 group was fed 1,000 ppm AQUANIN (300 ppm cysteamine hydrochloride) and standard shrimp feed. The G3 group was fed 3,000 ppm AQUANIN (900 ppm cysteamine hydrochloride) and standard shrimp feed. The Control group was fed standard shrimp feed only.

During the trial period, circulation of water was maintained, and water quality was kept at normal. At the end of this experiment, the total number of shrimp, the total body weight of the shrimp, and the average body weight of each shrimp were calculated.

Calculation of absolute body weight gain was performed by taking the average final body weight of each shrimp and subtracting this value from the average initial body weight of each shrimp.

Calculation of relative body weight gain was performed by dividing the average absolute body weight gain by the average initial body weight and multiplying this value by 100.

In the following Table 2, the data suggests that 500 ppm of AQUANIN presented the best growth promoting effect when compared against the other groups. The G1 group demonstrated: that 90% of the shrimp were alive at the end of the trial session, 11.95% increase in absolute body weight gain, and 6.25% improvement in food conversion ratio (FCR) after 42 days of treatment. The G2 and G3 groups, which were administered 1,000 ppm and 3,000 ppm of AQUANIN, respectively, also demonstrated some growth within the 42 day trial period. Specifically, the G2 and G3 groups demonstrated a 7.54-8.45% increase in absolute body weight gain and a 3.41-4.54% improvement in FCR.

| Table 2—Effect of AQUANIN on Juvenile Shrimp (*Penaeus vannamei*) Growth. | | | | |
|---|---|---|---|---|
| | 500 ppm Aquanin | 1,000 ppm Aquanin | 3,000 ppm Aquanin | Control |
| No. of shrimp | 90 | 90 | 90 | 90 |
| Average initial body weight (g/shrimp) | 0.47±0.02 | 0.46±0.02 | 0.46±0.02 | 0.452±0.02 |
| Average final body weight(g/shrimp) | 4.03±0.18 | 3.91±0.22 | 3.88±0.66 | 3.63±0.28 |
| Absolute body weight gain(g/shrimp) | 3.5±0.16 | 3.45±0.19 | 3.42±0.54 | 3.18±0.24 |
| Improvement in absolute body weight gain (%) | 11.95 | 8.45 | 7.54 | ---- |
| Relative body weight gain(%) | 773.80±54.03 | 749.65±18.36 | 749.24±40.85 | 710.18±70.09 |
| Improvement in relative body weight gain (%) | 7.46 | 5.49 | 5.48 | — |
| Feed Conversion Ratio (FCR) | 1.65±0.09 | 1.70±0.09 | 1.68±0.06 | 1.76±0.11 |
| Improvement in FCR (%) | 6.25 | 3.41 | 4.54 | — |
| Average live shrimp (%) | 90.00±9.81 | 77.50±11.67 | 89.17±5.69 | 87.22±10.63 |

As illustrated in Table 2, the addition of 500 ppm AQUANIN produced the best growth promoting effect on the juvenile shrimp. Thus, the administration of a cysteamine compound, in accordance with the subject invention, can help promote shellfish (in particular shrimp) health, including promotion of shellfish growth, improvement of shellfish feed conversion, enhancing shellfish longevity, and increasing shellfish body weight.

## Claims

1. A method for accelerating and/or augmenting shellfish growth, or enhancing shellfish fertility, which comprises administering to shellfish an effective amount of a cysteamine compound selected from cysteamine and salts thereof, and shellfish feed.

2. The method of claim 1, wherein the shellfish is selected from turtles, sea urchins, sea cucumbers, and jellyfish.

3. The method of claim 1, wherein the shellfish is selected from prawns, shrimp, crawfish, crayfish, crabs, lobsters, abalone, clams, mussels, oysters, scallops, octopi, squid, and snails.

4. The method of claim 3, wherein the shellfish is shrimp and the shellfish feed is shrimp feed.

5. The method of claim 4, wherein the effective amount of the cysteamine compound is 150 ppm of cysteamine hydrochloride.

6. The method of claim 1, further comprising administering to the shellfish, concurrently with the cysteamine compound, other known compounds used to improve shellfish health.

7. The method of claim 6, wherein the other known compounds are selected from microbicides, antimicrobials and vaccines.

8. The method of claim 7, wherein the other known compounds are selected from formalin, albendazole, cypermethrin, deltamethrin, hydrogen peroxide, teflubenzuron, oxytetracycline, alkyltrimethylammonium calcium oxytetracycline, bicozamycin benzoate, cyanphenicol, doxycycline, florofenicol, josamycin, kitasamycin, lincomycin, myroxacin, nalidixic acid, phosphomycin, spiramycin, sulfadimethoxine-ormetoprim, sulfamerazine, *Vibrio parahaemolyticus* (vibrogen-S) vaccine, penaeid multivalent bacterin (P.M.B. vaccine), and *Vibrio* sp. bacterin.

9. The method of any preceding claim, for enhancing shellfish fertility.

10. A composition comprising a cysteamine compound selected from cysteamine and salts thereof, and a shellfish feed, for improving shellfish health.

11. The composition of claim 10, wherein the shellfish feed is shrimp feed.

12. The composition of claim 10, which is in a form selected from liquid, aqueous mixture, aqueous emulsion, solid carrier, and solid substrate.

13. The composition of claim 10, comprising 0.1% to 95% of the cysteamine compound.

14. The composition of claim 10, to improve shellfish immune response.

15. The composition of claim 14, wherein the shellfish is oyster and the improved immune response is to diseases and pathogens selected from Oyster Velar Virus Disease (OVVD); Gill Disease of Portuguese Oysters; Haemocytic Infection Virus Disease of Oysters; Herpes-Type Virus Disease of Oysters; Extracellular Giant "Rickettsiae" of Oysters; *Perkinsus marinus* ("Dermo" Disease) of Oysters; *Perkinsus* sp. of European Flat Oysters; Kidney Coccidia of Oysters; *Minchinia armoricana* of Oysters; Marteiliosis (Aber disease) of Oysters; *Marteilioides chungmuensis* of Oysters; *Bonamia ostreae* of Oysters; Oyster Egg Disease; Invasive Ciliates of Juvenile Oysters; *Mytilicola intestinalis* (Red Worm Disease) of Oysters; Haemocytic Neoplasia of Oysters; Juvenile Disease of Eastern Oysters; Viral Gametocytic Hypertrophy of Oysters; Nocardiosis of Oysters; *Mikrocytos machine* (Demnan Island Disease) of Oysters; *Haplosporidium nelsoni* (MSX) of Oysters; *Haplosporidium costale* (SSO) of Oysters; *Ostracoblabe implexa* (Shell Disease) of Oysters; Oyster Trematode Diseases; *Mytilicola orientalis* (Red Worm) of Oysters; Parasite Copepods on Oyster Gills; Pea Crabs in Oysters; Malpeque Disease of Oysters; Papova-Like Virus Infection of Pearl Oysters; Apicomplexan Parasite of New Zealand Oysters; *Haplosporidium* sp. of Pearl Oysters; *Marteilia sydneyi* of Oysters; *Marteilioides branchialis* of Oysters; *Bonamia exitiosus* (Bonamiasis of New Zealand Dredge Oysters); *Bonamia* sp. (Bonamiasis of Australian Oysters); *Mikrocytos roughleyi* (Australian Winter Disease) of Oysters; Microsporidiosis of Dredge Oysters; *Rikettsia*-like and *Chlamydia*-like Organisms of Oysters; *Vibrio* spp. (Larval and Juvenile Vibriosis) of Oysters; Hinge Ligament Disease of Juvenile Oysters; Digestive Tract Impaction of Larval Oysters; Gregarine Parasitism of Oysters; Hexamitiasis of Oysters; *Ancistrocoma*-like Ciliates of Oysters; *Sphenophyra*-like Ciliates of Oysters; Gill Trichodinids of Oysters; *Sirolpidium zoophthorum* (Larval Mycosis) of Oysters; Oyster Gill Turbellaria; Nematode Parasitism of Oysters; Shell-boring Polychaetes of Oysters; Shell-burroing Sponges of Oysters; and Pyramidellid Snails of Oysters

16. The method of claim 14, wherein the shellfish is mussel and the improved immune response is to diseases and pathogens selected from Virus-like Diseases of Mussels; Haplosporidian Infection of Mussels; *Marteilia refringens*/*maurini* of Mussels; *Steinhausia mytilovum* (Mussel Egg Disease); Phototrophic Endolity Invasion of Mussel Shells; *Proctoeces maculates* Trematode Disease of Mussels; Mussel Gill Turbellaria; *Mytilicola intestinalis* (Red Worm Disease) of Mussels; Kidney Coccidia of Mussels; Bucephalid Trematode Diseases of Mussels; *Mytilicola orientalis* (Red Worm) of Mussels; Pea Crabs in Mussels; Haemocytic Neoplasia of Mussels; *Mytilicola porrecta* (Red Worm) of Mussels; *Rickettsia*-like and *Chlamydia-*like Organisms of Mussels; Gregarine Parasitism of Mussels; Intracellular Ciliates of Mussels; *Phenophyra*-like Ciliates of Mussels; *Ancistrum mytili* Gill Ciliate of Mussels; Mycotic Periostracal Sloughing of Mussels; Trematode Metacercariae of Mussels; Parasitic copepods on Mussel Gills; Shell-boring Polychaetes of Mussels; and Shell-burrowing Sponges of Mussels.

17. The composition of claim 14, wherein the shellfish is clam or cockle and the improved immune response is to diseases and pathogens selected from Viral infections of Clams; Brown Ring Disease of Manila Clams; *Perkinsus* of Clams and Cockles; Haplosporidian Infection of Clams; Microsporidiosis of Clams; Amoeboflagellate Disease of Larval Geoduck Clams; *Mytilicola inestinalis* (Red Worm Disease) of Clams and Cockles; Nuclear Inclusion X (NIX) of Pacific Razor Clams; Kidney Coccidia of Clams; QPX (quahog parasite unknown) of Clams; *Mytilicola orientalis* (Red Worm) of Clams and Cockles; Pea Crabs in Clams and Cockles; Haemocytic Neoplasia of Clams; Gonadal Neoplasia of Clams; Endonucleobiotic Bacteria of Clams in Portugal; Mycoplasma-like Infection of Cockles; Cryptosporidiosis of Clams; *Marteilia*-like Parasite of Giant Clams; Amoebiasis of Cockles; *Rickettsia*-like and *Chlamydia*-like Organisms of Clams and Cockles; *Vibrio* spp. (Larval and Juvenile Vibriosis) of Clams; Hinge Ligament Disease of Juvenile Clams; Gregarine Parasitism of Clams and Cockles; *Sphenophyra*-like Ciliates of Clams and Cockles, *Ancistrocoma pelseneeri* and A. *myae* Ciliates of Clams; Gill Trichodina of Clams and Cockles; *Sirolpidium zoophthorum* (Larval Mycosis) of Clams; Turbellaria of Clams; Trematode Metacercariae of Clams and Cockles; Shell-boring Polychaetes of Clams; and Siphon Snails of Clams and Cockles.

18. The composition of claim 14, wherein the shellfish is scallop and the improved immune response is to diseases and pathogens selected from *Perkinsus* sp. of Japanese Scallops in Asia; Scallop Haplosporidian; *Marteilia* sp. of Scallops; Brood-pouch Copepod of Scallop Gills; Pea Crabs in Scallops; Intracellular Bacterial Disease of Scallops; Bacterial Abscess Lesions of Scallops; *Perkinsus qugwadi* (SPX) of Scallops; Kidney Coccidia of Scallops; *Perdinsus karlssoni* of Scallops; Scallop Protistan G; Microsporidiosis of Scallops; Trematode Metacercariae of Scallops; Virus-like Infection of Scallops; Chlamydiosis of Scallops; *Rickettsia*-like and *Chlamydia*-like Organisms of Scallops; *Vibrio* spp. (Larval Vibriosis) of Scallops; Gregarine Parasitism of Scallops; Gill Trichodinids of Scallops; Scallop Gill Turbellaria; Nematode Parastitism of Scallops; Shell-boring Polychaetes of Scallops; and Shell-burrowing Sponges of Scallops.

19. The method of claim 14, wherein the shellfish is abalone and the improved immune response is to diseases and pathogens selected from Kidney Coccidia of Abalone; Sabellid Polychaete Infestation of Disease in Abalone; *Labyrinthuloides haliotidis* of Abalone; Amyotrophia of Abalone; Blister Disease of Cultured Abalone; Withering Syndrome of Abalone; *Perkinsus olseni* of Abalone; Haplosporidian parasite of Abalone; Fungal Disease of Abalone; Nematode Parasitism of Abalone; Bacterial Diseases of Abalone; Ciliates Associated with Abalone; Trematode Metacercariae of Abalone; and Shell-borrowing Polychaetes of Abalone.

20. The composition of claim 14, wherein the shellfish is sea urchin and the improved immune response is to diseases and pathogens selected from Namatode Parasitism of Sea Urchin; Bald-Sea-Urchin Disease; *Paramoeba invadens* of Sea Urchins; Spotted gonad Disease of Sea Urchins; Black Sea Urchin Plague; Trematode Metacercariae of Sea Urchins; and Turbellarian Parasitism of Sea Urchins.

21. The composition of claim 14, wherein the shellfish is lobster and the improved immune response is to diseases and pathogens selected from *Paramoeba perniciosa* (Paramoebiasis) of Lobsters; Gaffkemia of Lobsters; *Anophryoides haemophila* (Ciliate Disease) of Lobsters; *Pseudocarcinonemetes homari* of Lobsters; Microsporidosis of Lobsters; *Hematodinium* sp. of Norway Lobster; *Carcinonemertes australiensis* of Lobsters; Parasitic Copepods of Lobsters; *vibrio* spp. (Juvenile Vibriosis) of Lobsters; Chitinolytic Bacterial Shell Disease of Lobsters; Gregarine Parasitism of Lobsters; *Lagenidium* sp. (Fungus Disease) of Lobsters; *Fusarium* sp. (Fungus or Burn Spot Disease) of Lobsters; *Haliphthoros* sp. (Fungus Disease) of Lobsters; Nematodes in Lobsters; Trematode Metacercariae in Lobsters; and Acanthocephalan Larvae in Lobsters.

22. The composition of claim 14, wherein the shellfish is shrimp or prawn and the improved immune response is to diseases and pathogens selected from Rickettsia-like Infection of Pandalid Shrimp; Protistan Pathogen of Pandalid Shrimp (SPP); *Sylon* (Rhizocephalan Disease) of Shrimp and Prawns; *Baculovirus penaei* (BP Virus Disease) of Penaeid Shrimp; Monodon Baculovirus (MBV) Disease of Penaeid Shrimp; Baculoviral Midgut-gland Necrosis (BMN) of Penaeid Shrimp; White Spot Syndrome Baculovirus Complex of Penaeid Shrimp; Hepatopancreatic Parvovirus (HPV) Disease of Shrimp and Prawns; Infectious Hypodermal and Haematopoietic Necrosis Virus (IHHNV) of Penaeid Shrimp; Lymphoidal Parvo-like Virus Disease of Penaeid Shrimp; Lymphoid Organ Vacuolization Virus (LOVV) of Penaeid Shrimp; Reo-like Virus (REO) Disease of Penaeid Shrimp; Taura Syndrome Virus of Penaeid Shrimp; Rhabdovirus Disease of American Penaeid Shrimp; Yellow-head Virus Disease (YHD) of Penaeid Shrimp; Rickettsial Infection of Penaeid Shrimp; Necrotizing Hepatopancreatic of Penaeid Shrimp; Mycobacteriosis of Penaeid Shrimp; *Vibrio penaeicida* of Cultured Kurama Prawns; Larval Bacterial Necrosis of Freshwater Shrimp; Haplosporidian Infections of Penaeid Shrimp; Gregarine Disease of Penaeid Shrimp; Ciliate Disease of Penaeid Shrimp; Gut and Nerve Syndrome (GNS) of Penaeid Shrimp; Larval Mid-cycle Disease (MCD) of Freshwater Shrimp; Red Disease of Penaeid Shrimp; *Vibrio* spp. *(Vibrio* Disease) of Cultured Shrimp; Chitinolytic Bacterial Shell Disease of Shrimp and Prawns; Filamentous Bacterial Disease of Shrimp and Prawns; Microsporidosis (Cotton Shrimp Disease) of Shrimp and Prawns; Larval Mycosis of Shrimp and Prawns; *Fusarium* sp. (Fungus Disease) of Shrimp and Prawns; Nematomorph Parasitism of Pandalid Shrimp; and Black Gill Syndrome of Shrimp and Prawns.

23. The composition of claim 14, wherein the shellfish is crab and the improved immune response is to diseases and pathogens selected from Viral Diseases of Crabs; Rickettsia and Chlamydia of Crabs; Haplosporidosis of Crabs; *Paramoeba perniciosa* (Grey Crab Disease); *Hematodinium perezi* and *Hematodinium* sp. of Atlantic Crabs; Chitinolytic Fungal Disease (Black Mat Syndrome) of Crabs; *Carcinonemertes* spp. of Crabs; *Mesanophrys* spp. (Ciliate Disease) of Crabs; *Hematodinium* sp. (Bitter Crab Disease); Rhizocephalan Parasites of Crabs; *Hematodinium* spp. of Crabs in Australia; Chitinolytic Bacterial Shell Disease of Crabs; Microsporidosis of Crabs; Trematode Metacercariae of Crabs; Acanthocephalan Parasitism of Crabs; Nematomorph Parasitism of Crabs; and *Lagendium* spp. (Fungus Disease) of Crabs.

24. The composition of claim 14, wherein the shellfish is crayfish and the improved immune response is to diseases and pathogens selected from *Psorospermium* spp. (Protozoan Disease) of European Crayfish; Therlohaniasis of Crayfish; Burn Spot Disease (Fungus Disease) of Crayfish; Crayfish Plague (Fungus Disease); Baculovirus of Blue Crayfish; Rickettsia of Crayfish; *Nocardia* sp. (Bacterial Disease) Crayfish; *Proteus* or *Pseudomonas* Bacterial Septicaemia of Crayfish; Chitinolytic Bacterial Shell Disease of Crayfish; *Psorospermium* sp. (Protozoan Infection) of American Crayfish; *Saprolegnia* spp. (Fungus Disease) of Crayfish; Trematodes in Crayfish; Turbellaria Infestation of Crayfish; and Branchiobdellida Annelid Parasitism of Crayfish.

## Patentansprüche

1. Verfahren zum Beschleunigen und/oder Steigern des Wachstums von Schalentieren, oder Erhöhen der Fruchtbarkeit von Schalentieren, welches das Verabreichen einer wirksamen Menge einer Cysteamin-Verbindung, ausgewählt aus Cysteamin und Salzen davon, und von Schalentierfutter an Schalentiere umfasst.

2. Verfahren nach Anspruch 1, wobei die Schalentiere ausgewählt sind aus Schildkröten, Seeigeln, Seegurken und Quallen.

3. Verfahren nach Anspruch 1, wobei die Schalentiere ausgewählt sind aus Garnelen, Shrimps, Langusten (crawfish), Süßwasserkrebsen (crayfish), Krabben, Hummern, Abalone, essbaren Muscheln bzw. Venusmuscheln (clams), Muscheln (mussels), Austern, Kammmuscheln, Kraken, zehnarmigen Tintenfischen bzw. Kalmaren und Schnecken.

4. Verfahren nach Anspruch 3, wobei es sich bei den Schalentieren um Shrimps handelt und das Schalentierfutter Shrimpsfutter ist.

5. Verfahren nach Anspruch 4, wobei die wirksame Menge der Cysteamin-Verbindung 150 ppm Cysteamin-Hydrochlorid beträgt.

6. Verfahren nach Anspruch 1, außerdem umfassend das Verabreichen von anderen bekannten Verbindungen, die zum Verbessern der Gesundheit von Schalentieren verwendet werden, gleichzeitig mit der Cysteamin-Verbindung an die Schalentiere.

7. Verfahren nach Anspruch 6, wobei die anderen bekannten Verbindungen ausgewählt sind aus Mikrobiziden, antimikrobiellen Wirkstoffen und Impfstoffen.

8. Verfahren nach Anspruch 7, wobei die anderen bekannten Verbindungen ausgewählt sind aus Formalin, Albendazol, Cypermethrin, Deltamethrin, Wasserstoffperoxid, Teflubenzuron, Oxytetracyclin, Alkyltrimethylammonium-calcium-oxytetracyclin, Bicozamycinbenzoat, Cyanphenicol, Doxycyclin, Florfenicol, Josamycin, Kitasamycin, Lincomycin, Myroxacin, Nalidixinsäure, Phosphomycin, Spiramycin, Sulfadimethoxin-Ormetoprim, Sulfamerazin, *Vibrio parahaemolyticus* (Vibrogen-S)-Impfstoff, multivalentem Penaeiden-Bakterienimpfstoff (P.M.B.-Impfstoff) und *Vibrio* sp.-Bakterienimpfstoff.

9. Verfahren nach einem vorangehenden Anspruch zum Erhöhen der Fruchtbarkeit von Schalentieren.

10. Zusammensetzung, umfassend eine Cysteamin-Verbindung, ausgewählt aus Cysteamin und Salzen davon, und ein Schalentierfutter, zum Verbessern der Gesundheit von Schalentieren.

11. Zusammensetzung nach Anspruch 10, wobei das Schalentierfutter Shrimpsfutter ist.

12. Zusammensetzung nach Anspruch 10, welche in einer Form vorliegt, die ausgewählt ist aus einer Flüssigkeit, einer wässrigen Mischung, einer wässrigen Emulsion, einem festen Träger und einem festen Substrat.

13. Zusammensetzung nach Anspruch 10, umfassend 0,1 % bis 95 % der Cysteamin-Verbindung.

14. Zusammensetzung nach Anspruch 10 zum Verbessern der Immunantwort der Schalentiere.

15. Zusammensetzung nach Anspruch 14, wobei es sich bei den Schalentieren um Austern handelt und die verbesserte Immunantwort gegen Krankheiten und Pathogene gerichtet ist, die ausgewählt sind aus Oyster Velar Virus-Krankheit (Oyster Velar Virus Disease (OVVD)); Kiemenkrankheit von portugiesischen Austern; hämozytischer Infektionsviruskrankheit von Austern; Herpes-Typ-Viruskrankheit von Austern; extrazellulären Riesen-"Rickettsien" von Austern; *Perkinsus marinus* ("Dermo"-Krankheit) von Austern; *Perkinsus* sp. von europäischen Flachaustern; Nierenkokzidien von Austern; *Minchinia armoricana* von Austern; Marteiliosis (Aber-Krankheit) von Austern; *Marteilioides chungmuensis* von Austern; *Bonamia ostreae* von Austern; Austerneier-Krankheit; invasiven Ziliaten von juvenilen Austern; *Mytilicola intestinalis* (Rotwurm-Krankheit) von Austern; hämozytischer Neoplasie von Austern; juveniler Krankheit von östlichen Austern; viraler gametozytischer Hypertrophie von Austern; Nocardiose von Austern; *Mikrocytos machine* (Denman Insel-Krankheit) von Austern; *Haplosporidium nelsoni* (MSX) von Austern; *Haplosporidium costale* (SSO) von Austern; *Ostracoblabe implexa* (Schalen-Krankheit) von Austern; Austern-Trematoden-Krankheiten; *Mytilicola orientalis* (Rotwurm) von Austern; parasitische Kopepoden an Austernkiemen; Erbsenkrabben in Austern; Malpeque-Krankheit von Austern; Papova-artige Virusinfektion von Perlaustern; Apicomplexa-Parasit von neuseeländischen Austern; *Haplosporidium* sp. von Perlaustern; *Marteilia sydneyi* von Austern; *Marteilioides branchialis* von Austern; *Bonamia exitiosus* (Bonamiase von neuseeländischen Dredge-Austern); *Bonamia* sp. (Bonamiase von australischen Austern); *Mikrocytos roughleyi* (australische Winter-Krankheit) von Austern; Microsporidiose von Dredge-Austern; *Rikettsia*-artigen und *Chlamydia*-artigen Organismen von Austern; *Vibrio* spp. (larvale und juvenile Vibriose) von Austern; Scharnierband-Krankheit von juvenilen Austern; Verdauungstraktbefall von Austern-Larven; Gregarine-Parasitismus von Austern; Hexamitiase von Austern; *Ancistrocoma*-artigen Ziliaten von Austern; *Sphenophyra-*artigen Ziliaten von Austern; Kiemen-Trichodiniden von Austern; *Sirolpidium zoophthorum* (Larvenmykose) von Austern; Austernkiemen-Turbellaria; Nematoden-Parasitismus von Austern; schalenbohrenden Polychäten von Austern; schalenbohrenden Schwämmen von Austern; und Pyramidelliden-Schnecken von Austern.

16. Verfahren nach Anspruch 14, wobei es sich bei den Schalentieren um Muscheln handelt und die verbesserte Immunantwort gegen Krankheiten und Pathogene gerichtet ist, die ausgewählt sind aus virusartigen Krankheiten von Muscheln; Haplosporidien-Infektion von Muscheln; *Marteilia refringens*/*maurini* von Muscheln; *Steinhausia mytilovum* (Muscheleier-Krankheit); fototropher Endolity-Invasion von Muschelschalen; *Proctoeces maculates*-Trematoden-Krankheit von Muscheln; Muschelkiemen-Turbellaria; *Mytilicola intestinalis* (Rotwurm-Krankheit) von Muscheln; Nierenkokzidien von Muscheln; Bucephaliden-Trematoden-Krankheiten von Muscheln; *Mytilicola orientalis* (Rotwurm) von Muscheln; Erbsenkrabben in Muscheln; hämozytischer Neoplasie von Muscheln; *Mytilicola porrecta* (Rotwurm) von Muscheln; *Rickettsia-*artigen und *Chlamydia*-artigen Organismen von Muscheln; Gregarine-Parasitismus von Muscheln; intrazellulären Ziliaten von Muscheln; *Phenophyra*-artigen Ziliaten von Muscheln; *Ancistrum mytili*-Kiemenziliaten von Muscheln; mykotischer Periostrakum-Ablösung von Muscheln; Trematoden-Metazerkarien von Muscheln; parasitischen Kopepoden an Muschelkiemen; schalenbohrenden Polychäten von Muscheln; und schalenbohrenden Schwämmen von Muscheln.

17. Zusammensetzung nach Anspruch 14, wobei es sich bei den Schalentieren um Venusmuscheln (clam) oder Herzmuscheln (cockle) handelt und die verbesserte Immunantwort gegen Krankheiten und Pathogene gerichtet ist, die ausgewählt sind aus Virusinfektionen von Venusmuscheln; Braunring-Krankheit von Manila-Muscheln; Per*kinsus* von Venusmuscheln und Herzmuscheln; Haplosporidien-Infektion von Venusmuscheln; Microsporidiose von Venusmuscheln; Amoeboflagellaten-Krankheit von Geoduck-Muschellarven; *Mytilicola intestinalis* (Rotwurm-Krankheit) von Venusmuscheln und Herzmuscheln; nukleärem Einschluss X (NIX) von pazifischen Rasiermessermuscheln (Nuclear Inclusion X (NIX) of Pacific Razor Clams); Nierenkokzidien von Venusmuscheln; QPX (unbekannter Quahog-Parasit) von Venusmuscheln; *Mytilicola orientalis* (Rotwurm) von Venusmuscheln und Herzmuscheln; Erbsenkrabben in Venusmuscheln und Herzmuscheln; hämozytischer Neoplasie von Venusmuscheln; gonadaler Neoplasie von Venusmuscheln; endonukleobiotischen Bakterien von Venusmuscheln in Portugal; Mykoplasma-artiger Infektion von Herzmuscheln; Cryptosporidiose von Venusmuscheln; *Marteilia-*artigem Parasit von Riesenvenusmuscheln; Amoebiase von Herzmuscheln; *Rickettsia*-artigen und *Chlamydia*-artigen Organismen von Venusmuscheln und Herzmuscheln; *Vibrio* spp. (larvale und juvenile Vibriose) von Venusmuscheln; Scharnierband-Krankheit von juvenilen Venusmuscheln; Gregarine-Parasitismus von Venusmuscheln und Herzmuscheln; *Sphenophyra-*artigen Ziliaten von Venusmuscheln und Herzmuscheln, *Ancistrocoma pelseneeri* und *A. myae-*Ziliaten von Venusmuscheln; Kiemen-Trichodinen von Venusmuscheln und Herzmuscheln; *Sirolpidium zoophthorum* (Larvenmykose) von Venusmuscheln; Turbellaria von Venusmuscheln; Trematoden-Metazerkarien von Venusmuscheln und Herzmuscheln; schalenbohrenden Polychäten von Venusmuscheln; und Sipho-Schnecken von Venusmuscheln und Herzmuscheln.

18. Zusammensetzung nach Anspruch 14, wobei es sich bei den Schalentieren um Kammmuscheln handelt und die verbesserte Immunantwort gegen Krankheiten und Pathogene gerichtet ist, die ausgewählt sind aus *Perkinsus* sp. von japanischen Kammmuscheln in Asien; Kammmuschel-Haplosporidien; *Marteilia* sp. von Kammmuscheln; Brutbeutel-Kopepoden von Kammmuschelkiemen; Erbsenkrabben in Kammmuscheln; intrazellulärer Bakterienkrankheit von Kammmuscheln; bakteriellen Abszessläsionen von Kammmuscheln; *Perkinsus qugwadi* (SPX) von Kammmuscheln; Nierenkokzidien von Kammmuscheln; *Perdinsus karlssoni* von Kammmuscheln; Kammmuschel-Protistan G; Microsporidiose von Kammmuscheln; Trematoden-Metazerkarien von Kammmuscheln; virusartiger Infektion von Kammmuscheln; Chlamydiose von Kammmuscheln; *Rickettsia*-artigen und *Chlamydia*-artigen Organismen von Kammmuscheln; *Vibrio* spp. (Larvenvibriose) von Kammmuscheln; Gregarine-Parasitismus von Kammmuscheln; Kiemen-Trichodiniden von Kammmuscheln; Kammmuschelkiemen-Turbellaria; Nematoden-Parasitismus von Kammmuscheln; schalenbohrenden Polychäten von Kammmuscheln; und schalenbohrenden Schwämmen von Kammmuscheln.

19. Verfahren nach Anspruch 14, wobei es sich bei den Schalentieren um Abalone handelt und die verbesserte Immunantwort gegen Krankheiten und Pathogene gerichtet ist, die ausgewählt sind aus Nierenkokzidien von Abalone; Sabellid-Polychätenbefall-Krankheit in Abalone; *Labyrinthuloides haliotidis* von Abalone; Amyotrophie von Abalone; Blasenkrankheit von kultivierten Abalone; Welke-Syndrom (withering syndrome) von Abalone; *Perkinsus olseni* von Abalone; Haplosporidien-Parasit von Abalone; Pilzkrankheit von Abalone; Nematoden-Parasitismus von Abalone; Bakterienkrankheiten von Abalone; Ziliaten in Zusammenhang mit Abalone; Trematoden-Metazerkarien von Abalone; und schalenbohrenden Polychäten von Abalone.

20. Zusammensetzung nach Anspruch 14, wobei es sich bei den Schalentieren um Seeigel handelt und die verbesserte Immunantwort gegen Krankheiten und Pathogene gerichtet ist, die ausgewählt sind aus Nematoden-Parasitismus des Seeigels; Seeigel-Glatzenkrankheit (Bald-Sea-Urchin Disease); *Paramoeba invadens* von Seeigeln; Gefleckte-Gonaden-Krankheit von Seeigeln; schwarze Seeigelpest (Black Sea Urchin Plague); Trematoden-Metazerkarien von Seeigeln; und Turbellaria-Parasitismus von Seeigeln.

21. Zusammensetzung nach Anspruch 14, wobei es sich bei den Schalentieren um Hummer handelt und die verbesserte Immunantwort gegen Krankheiten und Pathogene gerichtet ist, die ausgewählt sind aus *Paramoeba perniciosa* (Paramoebiase) von Hummern; Gaffkemia von Hummern; *Anophryoides haemophila* (Ziliaten-Krankheit) von Hummern; *Pseudocarcinonemertes homari* von Hummern; Microsporidose von Hummern; *Hematodinium* sp. vom norwegischen Hummer; *Carcinonemertes australiensis* von Hummern; parasitischen Kopepoden von Hummern; *Vibrio* spp. (juvenile Vibriose) von Hummern; chitinolytischer bakterieller Schalen-Krankheit von Hummern; Gregarine-Parasitismus von Hummern; *Lagenidium* sp. (Pilzkrankheit) von Hummern; *Fusarium* sp. (Pilz- oder Brandfleckenkrankheit) von Hummern; *Haliphthoros* sp. (Pilzkrankheit) von Hummern; Nematoden in Hummern; Trematoden-Metazerkarien in Hummern; und Acanthocephalus-Larven in Hummern.

22. Zusammensetzung nach Anspruch 14, wobei es sich bei den Schalentieren um Shrimps oder Garnelen handelt und die verbesserte Immunantwort gegen Krankheiten und Pathogene gerichtet ist, die ausgewählt sind aus einer Rickettsien-artigen Infektion von Pandaliden-Shrimps; Protistan-Pathogen von Pandaliden-Shrimps (SPP); *Sylon* (Rhizocephala-Krankheit) von Shrimps und Garnelen; *Baculovirus penaei* (BP-Virus-Krankheit) von Penaeiden-Shrimps; Monodon Baculovirus (MBV)-Krankheit von Penaeiden-Shrimps; baculoviraler Mitteldarmdrüsen-Nekrose (BMN) von Penaeiden-Shrimps; Weißflecken-Syndrom-Baculovirus-Komplex von Penaeiden-Shrimps; hepatopankreatischer Parvovirus (HPV)-Krankheit von Shrimps und Garnelen; infektiösem hypodermalen und hämatopoietischen Nekrose-Virus (IHHNV) von Penaeiden-Shrimps; lymphoider Parvo-artiger Viruskrankheit von Penaeiden-Shrimps; lymphoidem Organvakuolisierungs-Virus (LOVV) von Penaeiden-Shrimps; Reo-artiges Virus (REO)-Krankheit von Penaeiden-Shrimps; Taura-Syndrom-Virus von Penaeiden-Shrimps; Rhabdovirus-Krankheit von amerikanischen Penaeiden-Shrimps; Gelbkopf-Virus-Krankheit (YHD) von Penaeiden-Shrimps; Rickettsien-Infektion von Penaeiden-Shrimps; nekrotisierender hepatopankreatischer Krankheit von Penaeiden-Shrimps; Mykobakteriose von Penaeiden-Shrimps; *Vibrio penaeicida* von kultivierten Kurama-Garnelen; bakterieller Nekrose von Süßwasser-Shrimp-Larven; Haplosporidien-Infektionen von Penaeiden-Shrimps; Gregarine-Krankheit von Penaeiden-Shrimps; Ziliat-Krankheit von Penaeiden-Shrimps; Darm-und-Nerven-Syndrom (GNS) von Penaeiden-Shrimps; Larven-Mittzyklus-Krankheit von Süßwasser-Shrimps (Larval Mid-cycle Disease (MCD) of Freshwater Shrimp); Rot-Krankheit von Penaeiden-Shrimps; *Vibrio* spp. (*Vibrio*-Krankheit) von kultivierten Shrimps; chitinolytischer bakterieller Schalenkrankheit von Shrimps und Garnelen; Filamentöse-Bakterien-Krankheit von Shrimps und Garnelen; Microsporidose (Baumwoll-Shrimp-Krankheit) von Shrimps und Garnelen; Larven-Mykose von Shrimps und Garnelen; *Fusarium* sp. (Pilzkrankheit) von Shrimps und Garnelen; nematomorphem Parasitismus von Pandaliden-Shrimps; und Schwarzkiemen-Syndrom von Shrimps und Garnelen.

23. Zusammensetzung nach Anspruch 14, wobei es sich bei den Schalentieren um Krabben handelt und die verbesserte Immunantwort gegen Krankheiten und Pathogene gerichtet ist, die ausgewählt sind aus Viruskrankheiten von Krabben; Rickettsien und Chlamydien von Krabben; Haplosporidose von Krabben; *Paramoeba perniciosa* (Graue-Krabbe-Krankheit, Gray Crab Disease); *Hematodinium perezi* und *Hematodinium* sp. von atlantischen Krabben; chitinolytischer Pilzkrankheit (Black Mat-Syndrom) von Krabben; *Carcinonemertes* spp. von Krabben; *Mesanophrys* spp. (Ziliaten-Krankheit) von Krabben; *Hematodinium* sp. (Bitterkrabbenkrankheit); Rhizocephala-Parasiten von Krabben; *Hematodinium* spp. von Krabben in Australien; chitinolytischer bakterieller Schalenkrankheit von Krabben; Microsporidose von Krabben; Trematoden-Metazerkarien von Krabben; Acanthocephala-Parasitismus von Krabben; nematomorphem Parasitismus von Krabben; und *Lagendium* spp. (Pilzkrankheit) von Krabben.

24. Zusammensetzung nach Anspruch 14, wobei es sich bei den Schalentieren um Süßwasserkrebse handelt und die verbesserte Immunantwort gegen Krankheiten und Pathogene gerichtet ist, die ausgewählt sind aus *Psorospermium* spp. (Protozoen-Krankheit) von europäischen Süßwasserkrebsen; Therlohaniase von Süßwasserkrebsen; Brandfleckenkrankheit (Pilzkrankheit) von Süßwasserkrebsen; Krebspest (Pilzkrankheit); Baculovirus des blauen Floridakrebses; Rickettsien von Süßwasserkrebsen; *Nocardia* sp. (Bakterien-Krankheit) von Süßwasserkrebsen; *Proteus* oder *Pseu*domonas-Bakterien-Septikämie von Süßwasserkrebsen; chitinolytische bakterielle Schalen-Krankheit von Süßwasserkrebsen; *Psorospermium* sp. (Protozoen-Infektion) von amerikanischen Süßwasserkrebsen; *Saprolegnia* spp. (Pilzkrankheit) von Süßwasserkrebsen; Trematoden in Süßwasserkrebsen; Turbellaria-Befall von Süßwasserkrebsen; und Branchiobdellida Anneliden-Parasitismus von Süßwasserkrebsen.

## Revendications

1. Procédé pour accélérer et/ou augmenter la croissance des mollusques et crustacés, ou pour amplifier la fertilité des mollusques et crustacés, qui comprend l'administration à un mollusque ou crustacés, d'une quantité efficace d'un composé cystéamine, choisi parmi la cystéamine et ses sels, et de la nourriture pour mollusques et crustacés.

2. Procédé selon la revendication 1, où le mollusque ou crustacé est choisi parmi les tortues de mer, les oursins, les concombres de mer et les méduses.

3. Procédé selon la revendication 1, où le mollusque ou crustacé est choisi parmi les crevettes roses, les crevettes, les langoustes, les écrevisses, les crabes, les homards, les ormeaux, les palourdes, les moules, les huitres, les coquilles Saint-Jacques, les poulpes, les calmars et les escargots.

4. Procédé selon la revendication 3, où le mollusque ou crustacé est choisi une crevette et la nourriture pour mollusque ou crustacé est de la nourriture pour crevette.

5. Procédé selon la revendication 4, où le la quantité efficace du composé cystéamine est de 150 ppm de chlorhydrate de cystéamine.

6. Procédé selon la revendication 1, comprenant en outre l'administration au mollusque ou crustacé, en même temps que le composé cystéamine, d'autres composés connus utilisés pour améliorer la santé des mollusques et crustacés.

7. Procédé selon la revendication 6, où les autres composés connus sont choisis parmi les microbicides, les antimicrobiens et les vaccins.

8. Procédé selon la revendication 7, où le les autres composés connus sont choisis parmi la formaline, l'albeneazole, la cyperméthrine, la deltaméthrine, le peroxyde d'hydrogène, le teflubenzuron, l'oxytétracycline, le sel d'alkyltriméthylammonium et de calcium de l'oxytétracycline, le benzoate de bicozamycine, le cyanphénicol, la doxycycline, le florofénicol, la josamycine, la kitasamycine, la lincomycine, la myroxacine, l'acide naldixique, la phosphomycine, la spiramycine, la sulfadiméthoxine-ormétoprim, la sulfamérazine, la vaccin *Vibrio parahaemolyticus* (vibrogène-S), bactérine multivalente pour pénéide (vaccin P.M.B.) et bactérine *Vibrio* sp.

9. Procédé selon l'une quelcoque des revendications précédentes, pour amplifier la fertilité des mollusques et crustacés.

10. Composition comprenant un composé cystéamine choisi parmi la cystéamine et ses sels, pour de la nourriture ou mollusque ou crustacé, pour améliorer la santé des mollusques et crustacés.

11. Composition selon la revendication 10, où la pour mollusque ou crustacé est de la nourriture pour crevette.

12. Composition selon la revendication 10, qui est sous forme sélectionnée parmi liquide, mélange aqueux, émulsion aqueuse, support solide et substrat solide.

13. Composition selon la revendication 10, comprenant 0,1% à 95% du composé cystéamine.

14. Composition selon la revendication 10, pour améliorer la réponse immune des mollusques et crustacés.

15. Composition selon la revendication 14, où le mollusque ou crustacé est une huitre et la réponse immune améliorée est contre les maladies et pathogènes choisis parmi la maladie due à l'Oyster Vela Virus (OVVD) ; la maladie des branchies des huitres portugaises ; la maladie du virus de l'infection des hémocytes de l'huitre ; la maladie du virus de type herpès des huitres ; « Rikettsie » extracellulaire de grande taille de l'huitre ; *Perkinsus marinus* (maladie « dermo ») des huitres ; *Perkinsus* sp. des huitres plates européennes ; coccidie rénale des huitres ; *Minchinia armoricana* des huitres ; la marteiliose des huitres ; *Marteilioides chungluensis* des huitres ; *Bonamia ostrae* des huitres ; la maladie des oeufs d'huitre ; les ciliés invasifs des jeunes huitres ; *Mytilicola intestinalis* (maladie de vers rouges) des huitres ; néoplasie des hémocytes des huitres ; maladie juvénile des huitres de l'est ; hypertrophie virale des gamétocytes des huitres ; nocardiose des huitres ; *Mikrocytos machine* (maladie de l'île de Denman) des huitres ; *Haplosporidium nelsoni* (MSX) des huitres ; *Haplosporidium costale* (SSO) des huitres ; *Ostracoblabe implexa* (maladie de la coquille) des huitres ; maladies des trématodes des huitres ; *Mytilicola orientalis* (vers rouges) des huitres ; les copépodes parasites sur les branchies des huitres ; les crabes petits pois des huitres ; la maladie Malpèque des huitres ; l'infection par le virus de type papova des huitres perlières ; le parasite apicomplexe des huitres de Nouvelle Zélande ; *Haplosporidium* sp. des huitres perlières ; *Marteilia sydneyi* des huitres ; *Marteilioides branchialis* des huitres ; *Bonamia exitosus* (bonamiasie des huitres de Nouvelle Zélande) ; *Bonamia* sp. (bonamiasie des huitres australienne) ; *Mikrocytos roughleyi* (maladie de l'hiver australien) des huitres ; la microsporidiose des huitres creuses ; des organismes de type rickettsie et de type chlamydiae des huitres ; *Vibrio* spp. (vibrioses larvaire et juvénile) des huitres ; maladie du ligament de charnière des huitres juvéniles ; l'impaction du tractus digestif des huitres larvaires ; grégarine parasite des huitres ; hexamatiase des huitres ; ciliés de type *Ancistrocoma* des huitres ; ciliés de type *Sphenophyra* des huitres ; trichodinide des branchies des huitres ; *Sirolpidium zoophtorum* (mycose larvaire) des huitres ; tuberllaria des branchies des huitres ; nématode parasite des huitres ; polychètes sur les coquille des huitres ; éponges sur les coquilles des huitres, et escargots pyramidéllide des huitres.

16. Procédé selon la revendication 14, où le mollusque ou crustacé est une moule et la réponse immune améliorée est contre les maladies et pathogènes choisis parmi les maladies de type virus des moules ; infection par Haplosporide des moules ; *Marteilia refringens*/*maurini* des moules ; *Steinhausia mytilovum* (maladie des oeufs de moules) ; l'infection phototrophe des coquilles de moule ; trématode *Proctoeces maculates* des moules ; turbellaria des branchies des moules ; *Mytilicola intestinalis* (maladie de vers rouges) des moules ; coccidie rénale des moules ; trématode bucéphalide des moues ; *Mytilicola orientalis* (vers rouge) des moules ; crabes petits pois des moules ; néoplasie de hémocytes des moules ; *Mytilicola porrecta* (ver rouge) des moules ; des organismes de type rickettsie et de type chlamydiae des moules ; grégarine parasite des moules ; ciliés intracellulaire des moules ; ciliés de type Sphénophyra des moules ; cilié *Ancistrum mytili* des branchies des moules ; ramollissement mycosique du pied de la moule ; trématode Metacercariae des moules ; copépodes parasites des branchies des moules ; polychètes sur la coquille des moules ; et éponges sur les coquilles des moules.

17. Procédé selon la revendication 14, où le mollusque ou crustacé est une palourde ou coque et la réponse immune améliorée est contre les maladies et pathogènes choisis parmi les infections virales des palourdes ; la maladie à anneau brun des palourdes de Manille ; Perkinsus des palourdes et coques ; infection par Haplosporide des palourdes ; microsporidiose des palourdes ; maladie par amiboflagellé des palourdes larvaires ; *Mytilicola intestinalis* (maladie de vers rouges) des palourdes et coques ; inclusion nucléaire X (NIX) des palourdes du Pacifique ; coccidie rénale des palourdes ; QPX (parasite quahog inconnu) des palourdes ; *Mytilicola orientalis* (vers rouge) des palourdes et coques ; crabes petits pois des palourdes et coques ; néoplasie de hémocytes des palourdes ; néoplasie des gonades des palourdes ; bactérie endonucléobiotique des palourdes du Portugal ; Infection de type mycoplasme des coques ; cryptosporidiose des palourdes ; parasite de type *Marteilia* des palourdes géantes ; amibiase des coques ; organismes de type rickettsie et de type chlamydiae des palourdes et coques ; *Vibrio* sp. (vibrioses larvaire et juvénile) des palourdes ; maladie du ligament de charnière des palourdes juvéniles ; grégarine parasite des palourdes et coques ; ciliés de type Sphénophyra des palourdes et coques ; ciliés *Ancistrocoma pelseneerie et A. myae* des palourdes ; des branchies des palourdes et coques ; *Sirolpidium zoophthorum* (mycose larvaire) des palourdes ; turbellaria des palourdes ; trématode *Metacercariae* des palourdes et coques ; polychètes sur la coquille des palourdes ; et escargots des palourdes et coques.

18. Procédé selon la revendication 14, où le mollusque ou crustacé est une coquille Saint-Jacques et la réponse immune améliorée est contre les maladies et pathogènes choisis parmi *Perkinsus* sp. de la coquille Saint-Jacques du Japon ; Haplosporidie de coquille Saint-Jacques ; *Marteilia* sp. de coquille Saint-Jacques ; copépode des branchies de coquille Saint-Jacques ; crabes petits pois de coquille Saint-Jacques ; maladie bactérienne intracellulaire de coquille Saint-Jacques ; lésions bactériennes de coquille Saint-Jacques ; *Perkinsus qugwadi* (SPX) des coquille Saint-Jacques ; coccidie rénale de coquille Saint-Jacques ; *Perdinsus karlssoni* de coquille Saint-Jacques ; protiste G de coquille Saint-Jacques ; microsporridiose de coquille Saint-Jacques ; trématode *Metacercariae* de coquille Saint-Jacques ; infection de type virus de coquille Saint-Jacques ; chlamydiose de coquille Saint-Jacques ; organismes de type rickettsie et de type chlamydiae de coquille Saint-Jacques ; *Vibrio* sp. (vibriose larvaire) de coquille Saint-Jacques ; grégarine parasite de coquille Saint-Jacques ; trichodinide des branchies de coquille Saint-Jacques ; tuberllaria de branchies de coquille Saint-Jacques ; nématode parasite de coquille Saint-Jacques ; polychètes de coquille Saint-Jacques, et éponges de coquille Saint-Jacques.

19. Procédé selon la revendication 14, où le mollusque ou crustacé est un ormeau et la réponse immune améliorée est contre les maladies et pathogènes choisis parmi coccidie rénale des ormeaux ; infection ou maladie par polychète sabellide des ormeaux ; *Labyrinthuloides haliotidis* des ormeaux ; amyotrophie des ormeaux ; maladie des cloques des ormeaux ; syndrome de dessèchement des ormeaux ; *Perkinsus olseni* des ormeaux ; parasite haplosporidie des ormeaux ; maladie fongique des ormeaux ; nématode parasite des ormeaux ; maladies bactériennes des ormeaux ; ciliés associés aux ormeaux ; trématode *Metacercariae* des ormeaux; et polychètes des ormeaux.

20. Procédé selon la revendication 14, où le mollusque ou crustacé est un oursin et la réponse immune améliorée est contre les maladies et pathogènes choisis parmi le nématode parasite de l'oursin ; la maladie de « l'oursin chauve » ; *Paramoeba invadens* de l'oursin ; la maladie des gonades de l'oursin ; la plague de l'oursin de Mer Noure ; le trématode *Metacercariae* de l'oursin ; et le parasite *Tuberllaria* de l'oursin.

21. Procédé selon la revendication 14, où le mollusque ou crustacé est un homard et la réponse immune améliorée est contre les maladies et pathogènes choisis parmi *Paramoeba perniciosa* (paramibiase) du homard ; la gaffkémie du homard ; *Anophryoides haemophila* (cilié) du homard ; *Pseudocarcinonemetes homari* du homard ; la microsporidose du homard ; *Hematodinium* sp. du homard de Norvège ; *Carcinonemertes australiensis* du homard ; des copépodes parasites du homard ; *Vibrio* sp. (vibriose juvénile) du homard ; la maladie bactérienne chitinolytique du homard ; la grégarine parasite du homard ; *Lagenidium* sp. (maladie fongique) du homard ; *Fusarium* sp. (maladie fondique ou tâche brûlée) du homard ; *Haliphthoros* sp. (maladie fongique) du homard ; des nématodes du homard ;, le trématode *Metacercariae* du homard ; et les larves *Acanthocephalan* du homard.

22. Procédé selon la revendication 14, où le mollusque ou crustacé est une crevette rose ou grise et la réponse immune améliorée est contre les maladies et pathogènes choisis parmi une infection de type rickettsie de la crevette striée ; un pathogène protiste de la crevette striée (SPP) ; Sylon (maladie rhizocéphale) des crevettes ; *Baculovirus penaei* (virus BP) de la crevette pénéide ; *Baculovirus monodon* (MBV) de la crevette pénéide ; la nécrose baculovirale de la glande moyenne (BMN) de la crevette pénéide ; le complexe baculovirus du syndrome de la tache blanche de la crevette pénéide ; la parvovirus hépatopancréatique (HPV) des crevettes ; le virus infectieux de nécrose hypodermique et hématopoïétique (IHHNV) de la crevette pénéide ; le virus de type parvo lymphoïde de la crevette pénéide ; le virus de vacuolisation de l'organe lymphoïde (LOVV) de la crevette pénéide ;le virus de type réo (REO) de la crevette pénéide ; le virus du syndrome Taura de la crevette pénéide ; la maladie du rhabdovirus de la crevette pénéide américaine ; la maladie du virus de tête jaune (YHD) de la crevette pénéide ;; une infection par rickattsie de la crevette pénéide ; la nécrose hépatopancréatique de la crevette pénéide ; la mycobactériose de la crevette pénéide ; *Vibrio penaeicida* des crevette Kurama en élevage ; la nécrose bactérienne larvaire de la crevette d'eau douce ; les infections par haplosporidie de la crevette pénéide ; la maladie de la grégarine de la crevette pénéide ; la maladie ciliée de la crevette pénéide ; le syndrome intestin et nerf (GNS) de la crevette pénéide ; la maladie de cycle moyen larvaire (MCD) de la crevette d'eau douce ; la maladie rouge de la crevette pénéide ; *Vibrio* spp. de la crevette d'élevage ; la maladie bactérienne chitinolytique des crevettes ; la maladie bactérienne filamenteuse des crevettes ; la microsporidose (Cotton Shrimp Disease) des crevettes ; la mycose larvaire des crevettes ; *Fusarium* sp. (maladie fongique) des crevettes ; le parasite nématomorphe de la crevette pandalide ; et le syndrome des branchies noires des crevettes.

23. Procédé selon la revendication 14, où le mollusque ou crustacé est un crabe et la réponse immune améliorée est contre les maladies et pathogènes choisis parmi les maladies virales des crabes ; les rickettsies et chlamydias des crabes ; l'haplosporidose des crabes ; *Paramoeba perniciosa* (maladie du crabe gris) ; *Hematodinium perezi* et *Hematodinium* sp. du crabe atlantique ; le maladie fongique chitinolytique (syndrome du mat noir) des crabes ; *Carcinonemertes* sp. des crabes ; *Mesanophrys* sp. (maladie ciliée) des crabes ; *Hematodinium* sp. (maladie du crabe amer) ; les parasites rhizocephalan des crabes ; *Hematodinium* sp. des crabes en Australie ; la maladie bactérienne chitinolytique des crabes ; la microsporidose des crabes ; le trématode *Metacercariae* des crabes ; le parasite *Acanthocephalan* des crabes ; un parasite nématomorphe des crabes et *Lagendium* sp. des crabes.

24. Procédé selon la revendication 14, où le mollusque ou crustacé est une langouste et la réponse immune améliorée est contre les maladies et pathogènes choisis parmi *Psorospermium* sp. (protozoaire) de la langouste européenne ; la therlohaniase de la langouste ; la maladie de la tache brulée (fongique) de la langouste ; la plague de la langouste ; le baculovirus de la langouste bleue ; la rickettsie de la langouste ; *Nocardia* sp. (maladie bactérienne) de la langouste ; une septicémie bactérienne Proteus ou *Pseudomonas* de la langouste ; la maladie bactérienne chitinolytique de la langouste ; *Psorospermium* sp. (infection protozoaire) de la langouste américaine ; *Saprolegnia* sp. (maladie fongique) de la langouste ; des trématodes de langouste ; une infection par *Turbellaria* de la langouste ; et le parasite annélide *Branchiobdellida* de la langouste.
